(19) Europäisches Patentamt
European Patent Office
Office européen des brevets

(11) Publication number: **0 430 234 A2**

# (12) EUROPEAN PATENT APPLICATION

(21) Application number: **90122842.9**

(51) Int. Cl.[5]: **C07C 227/04**, C07C 229/22

(22) Date of filing: **29.11.90**

(30) Priority: **29.11.89 JP 311519/89**

(43) Date of publication of application:
**05.06.91 Bulletin 91/23**

(84) Designated Contracting States:
**BE CH DE ES FR GB IT LI NL Bulletin**

(71) Applicant: **Kanegafuchi Chemical Industry Co., Ltd.**
**2-4 Nakanoshima 3-chome**
**Kita-ku Osaka-shi Osaka-fu(JP)**

(72) Inventor: **Takahashi, Satomi**
**1-13-13, Shinwadai, Tarumi-ku**
**Kobe-shi, Hyogo-ken(JP)**
Inventor: **Yanagida, Yoshifumi**
**2-63, Okihama-cho, Takasago-cho**
**Takasago-shi, Hyogo-ken(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

## (54) Process for the production of omega-amino-alpha-hydroxycarboxylic acid.

(57) An optically active ω-amino-α-hydroxycarboxylic acid of the formula:

$$HO-\underset{\displaystyle COOH}{\underset{|}{CH}}-(CH_2)_n-NH_2$$

wherein n is 1 or 2, which process comprises reacting an ω-ester of an α-amino acid with nitrous acid under an acidic condition to convert an amino group to an hydroxy group to obtain an ω-alkoxycarbonyl-α-hydroxycarboxylic acid, reacting the ω-alkoxycarbonyl-α-hydroxycarboxylic acid with ammonia to convert an alkoxycarbonyl group to an amide group to obtain an ω-aminocarbonyl-α-hydroxycarboxylic acid, and reacting the ω-aminocarbonyl-α-hydroxycarboxylic acid with an active halogen to convert the amido group to an amino group.

EP 0 430 234 A2

PROCESS FOR THE PRODUCTION OF OMEGA-AMINO-ALPHA-HYDROXYCARBOXYLIC ACID

## BACKGROUND OF THE INVENTION

### Field of the Invention

The present invention relates to a process for the production of an ω-amino-α-hydroxycarboxylic acid. More particularly, the present invention relates to a process for the production of an optically active ω-amino-α-hydroxycarboxylic acid from an ω-ester of an optically active α-amino acid.

### Description of the Related Art

An optically active ω-amino-α-hydroxycarboxylic acid is a useful intermediate in the synthesis of medicines. In particular, L-γ-amino-α-hydroxybutyric acid (L-AHBA) and L-β-amino-α-hydroxypropionic acid (L-AHPA) are useful as side-chain intermediates in the synthesis of semisynthetic aminoglycoside type antibiotics such as Amikacin and Isepamicin, respectively.

As processes for the production of L-AHBA, there are known a process comprising converting a β-aminocarbonyl group of L-asparagine to a cyano group and then reducing the cyano group to an aminomethylene group and separately diazotizing an α-amino group to form a hydroxyl group (see Japanese Patent Kokai Publication No. 100316/1979 and Bull. Chem. Soc. Jpn., 51, 3296 (1978)), and a process comprising diazotizing an α-amino group of L-glutamic acid, then cyclizing an a-hydroxyglutamic acid with dehydration to from γ-carboxy-γ-butyrolactone, aminating it to form glutaramic acid which is subjected to the Hofmann reaction (see Japanese Patent Kokai Publication No. 4019/1975).

As processes for the production of L-AHPA, there are known a process comprising diazotizing an α-amino group of L-asparagine to form a hydroxyl group and subjecting a β-aminocarbonyl group to the Hofmann reaction with active halogen (see Agr. Biol. Chem., 40, 1651 (1976)), a process comprising converting L-malic acid to a β-monoalkyl ester by utilizing a position selective hydrolysis of L-malic acid, amidating said ester to form a β-malamidic acid and then reacting malamidic acid with active halogen (see Japanese Patent Kokai publication No. 190093/1987), and a process which uses D-glucalamine (see Japanese Patent Kokai Publication No. 35152/1983).

However, in the conventional processes utilizing L-asparagine, L-malic acid and D-glucalamine, the starting materials are expensive and yields of the products are not satisfactory. In the process utilizing L-glutamic acid, while the starting material is easily available, the preparation of the intermediate γ-carboxy-γ-butyrolactone is troublesome. Therefore, this process is not a convenient process for the production of AHBA.

## SUMMARY OF THE INVENTION

An object of the present invention is to provide a novel process for the production of an optically active ω-amino-α-hydroxycarboxylic acid, which process is economically attractive and easily carried out with good efficiency.

According to the present invention, there is provided a process for the production of an optically active ω-amino-α-hydroxycarboxylic acid of the formula:

$$\underset{\displaystyle COOH}{HO-\underset{|}{C}H}-(CH_2)_n-NH_2 \qquad (IV)$$

wherein n is 1 or 2, which process comprises steps of:

(1) reacting an ω-ester of an α-amino acid of the formula:

$$NH_2-\underset{\displaystyle COOH}{\underset{|}{CH}}-(CH_2)_n-COOR \qquad (I)$$

wherein n is the same as defined above, and R is a $C_1$-$C_7$ alkyl group or an aralkyl group with nitrous acid under an acidic condition to convert an amino group to an hydroxy group to obtain an ω-alkoxycarbonyl-α-hydroxycarboxylic acid of the formula:

$$OH-\underset{\displaystyle COOH}{\underset{|}{CH}}-(CH_2)_n-COOR \qquad (II)$$

wherein R and n are the same as defined above,

(2) reacting the ω-alkoxycarbonyl-α-hydroxycarboxylic acid (II) with ammonia to convert an alkoxycarbonyl group to an amide group to obtain an ω-aminocarbonyl-α-hydroxycarboxylic acid of the formula:

$$OH-\underset{\displaystyle COOH}{\underset{|}{CH}}-(CH_2)_n-CONH_2 \qquad (III)$$

wherein n is the same as defined above, and

(3) reacting the ω-aminocarbonyl-α-hydroxycarboxylic acid (III) with an active halogen to convert the amido group to an amino group to obtain the ω-amino-α-hydroxycarboxylic acid (IV).

## DETAILED DESCRIPTION OF THE INVENTION

The optically active ω-ester of α-amino acid (I) is easily prepared by ω-monoesterifying optically active aspartic acid or glutamic acid or their salt with alkali metals (e.g. sodium salts, potassium salts, etc.) in an alcohol in the presence of an acid catalyst.

As the alcohol, methanol, ethanol, isopropanol, n-butanol and the like may be used. As the acid catalyst, sulfuric acid, hydrochloric acid, p-toluenesulfonic acid and the like may be used.

The conditions for ω-monoesterification are not critical and can be the same as those employed in conventional esterification. Through control of an amount of the acid catalyst (usually 1 to 2 equivalents), a reaction temperature (usually around room temperature) and a reaction time (usually about 20 hours), an yield of about 90 % is achieved. If desired, the produced ω-ester of α-amino acid is isolated and purified by a per se conventional method such as ion-exchange column chromatography, and used in the next step. Alternatively, the reaction solution as such is used in the next step, if desired, after neutralizing the solution and evaporation off the alcohol under reduced pressure.

The reaction of the resulting optically active ω-ester of α-amino acid with nitrous acid is preferably carried out in an acidic aqueous medium by gradually adding an nitrite to generate nitrous acid in the reaction system.

When a strong acid is used in a large amount, the ester tends to be hydrolyzed. Therefore, the strong acid such as hydrochloric acid or sulfuric acid is preferably used in an about equivalent amount to the ω-ester of α-amino acid. To suppress side reactions and increase a reaction rate, a weak acid, in particular acetic acid is preferably used.

The reaction temperature is usually from -5 to 40 °C, preferably from 0 to 30 °C, more preferably from 5 to 20 °C.

Any nitrite may be used to generate nitrous acid. Preferably, alkali metal salts of nitrous acid (e.g. $NaNO_2$) or alkaline earth metal salts of nitrous acid (e.g. $Ca(NO_2)_2$) are used.

Under the above preferred conditions, the ω-ester of α-amino acid can be converted to the ω-ester of α-hydroxycarboxylic acid in substantially a stoichiometric yield.

The conversion of the ω-ester of α-hydroxycarboxylic acid to the ω-amide of α-hydroxycarboxylic acid is carried out by using an aqueous ammonia or a mixture of ammonia and an alcohol at a temperature of from

0 to 50° C, Preferably around room temperature. An amount of ammonia is at least two equivalents to the ω-ester. Usually, the ammonia is used in a large excess amount. The resulting ω-amide of α-hydroxycarboxylic acid can be isolated and purified in the crystalline form by evaporating off the ammonia and contacting the reaction mixture with an anion exchange resin, although the reaction mixture as such may used in the next step after concentration under reduced pressure.

The conversion of the ω-amide of α-hydroxycarboxylic acid to the ω-amino-α-hydroxycarboxylic acid with the active halogen (the Hofmann reaction) is carried out by using the active halogen in 0.8 to 1.2 equivalents, preferably about 1.00 equivalent under a basic condition to convert the amido group to an isocyanate group and then heating the compound at a temperature of 40 to 100° C to effect decarboxylation.

Though the active halogen may be generated by adding halogen to an aqueous solution of an alkali metal hydroxide, an aqueous solution of sodium hypochlorite is preferably used since it is easily handled. Although the resulting ω-amino-α-hydroxycarboxylic acid may be purified and isolated by treating the reaction mixture with a cation resulting ω-amino-α-hydroxycarboxylic acid may be purified and isolated by treating the reaction mixture with a cation exchange resin, it is preferably purified by recrystallizing it from a combination of an alcohol and water while desalination.

The ω-amino-α-hydroxycarboxylic acid which is produced by the above reaction steps is optically pure. From the ω-ester of L-amino acid, L-ω-amino-α-hydroxycarboxylic aid is produced, and from the ω-ester of D-amino acid, D-ω-amino-α-hydroxycarboxylic acid is produced.

## PREFERRED EMBODIMENTS OF THE INVENTION

The present invention will be illustrated by following Examples.

### Reference Example 1

Preparation of 5-methyl L-glutamate (Ia)

In a mixture of methanol (300 ml) and conc. sulfuric acid (24.3 g), sodium L(+)-glutamate (hydrate) (30 g) was added and stirred at room temperature overnight. The analysis of the reaction mixture with high performance liquid chromatography (HPLC) revealed that 5-methyl L-glutamate (Ia) (23.2 g, purity of 89.9 %) was formed.

### Example 1

Preparation of L-2-hydroxyglutaramic acid (IIIa)

In a mixed solvent of water (50 ml) and acetic acid (50 ml), 5-methyl L-glutamate (Ia) (10 g, 62.05 mmol) was added. Then, to the mixture, a 40 % (w/w) aqueous solu tion of sodium nitrite (14.2 g) was added over about 5 hours while stirring and further stirred overnight.

From the reaction mixture, acetic acid was evaporated off under reduced pressure, and the concentrate was gradually poured in an ice-cooled 28 % aqueous ammonia. After pouring, the reaction solution was gradually warmed to room temperature and kept standing overnight, followed by evaporation under reduced pressure to obtain a syrup. Then, to the syrup, water (20 ml) was added to form a solution, and the solution was passed through a column containing Amberlite IR-120B resin ($H^+$ type, 200 ml) followed by washing with water. Acidic eluates having pH of not larger than 4.0 (about 1.0 1) were collected and evaporated under reduced pressure to obtain white crystalline L-2-hydroxyglutaramic acid (IIIa) (7.06 g, purity of 77.4 %). The acid (IIIa) was dissolved in ethanol (45 ml) by heating and cooled to crystallize it. The crystallized product was recovered by filtration and dried under vacuum.

The properties of L-2-hydroxyglutaramic acid were as follows:

Melting point: 119-120° C
$[\alpha]_D^{25}$ = +2.04° (c = 2.5 in water)
Thin layer silica gel chromatography (coloring with phosphomolybdic acid): Rf = 0.4
(ethanol:acetic acid:butanol:water = 10:2:2:1)

Preparation of L(-)-4-amino-2-hydroxybutyric acid

(IVa)

To an ice-cooled mixture of a 5 % aqueous solution of sodium hypochlorite (40.5 g) and a 2.5 N aqueous solution of sodium hydroxide (65.3 ml), L-2-hydroxyglutaramic acid (IIIa) obtained in the above step (4.0 g, 27.21 mmol) was added while stirring. Then the mixture was stirred at room temperature for 10 minutes and then at 70° C for 20 minutes and cooled. After the mixture was acidified to pH of 4.0 with conc. hydrochloric acid, it was adsorbed on Amberlite IR-120B resin ($H^+$ type, 400 ml), washed with water till pH of the washing liquid became neutral (pH = 5 to 6) and eluted with 1N aqueous ammonia.

The eluate was collected fraction by fraction, and the ninhydrine positive fractions were combined and evaporated under reduced pressure to obtain yellowish syrupy concentrate. The concentrate was dissolved in water (10 ml). Then, to the solution, ethanol (28 ml) was added to crystallize the product. The crystal was recovered by filtration and dried under vacuum to obtain L(-)-4-amino-2-hydroxybutyric acid (IVa) (2.49 g, purity of 76.9 %), which was recrystallized from water/ethanol.

The properties of L(-)-4-amino-2-hydroxybutyric acid were as follows:

Melting point: 208-209° C

$[\alpha]_D^{23}$ .5 = -28.5° (c = 2.5 in water)

$^1$H-NMR ($D_2O$) : $\delta$ (ppm) = 4.20-3.98 (1H, q), 3.23-2.93 (2H, t) and 2.23-1.70 (2H, m).

Example 2

The reaction mixture (500 ml) containing 5-methyl L-glutamate (224.7 mmol) which was produced in Reference Example 1 was neutralized to pH of 6.0 with a saturated aqueous solution of sodium bicarbonate. After evaporating off methanol under reduced pressure, water was added to a whole volume of 240 ml. To the mixture, acetic acid (60.7 g) was added and then a 40 % (w/w) aqueous solution of sodium nitrite (59.0 g) was added over about 5 hours while stirring at a temperature of 15 to 20° C, followed by stirring overnight. After evaporating off acetic acid under reduced pressure, the reaction mixture was acidified to pH of 2.0 with conc. hydrochloric acid and extracted with ethyl acetate (each 50 ml) five times. The combined organic layers were dried over magnesium sulfate, and ethyl acetate was evaporated off under reduced pressure to obtain a syrupy concentrate (41.6 g).

Then, the syrup was dissolved in water (50 ml) and poured in an ice-cooled 28 % aqueous ammonia. The mixture was gradually warmed to room temperature and kept standing overnight. The analysis of the reaction mixture with high performance liquid chromatography (HPLC) revealed that L-2-hydroxyglutaramic acid (IIIa) (24.6 g, 167.34 mmol) was formed. ice-cooled mixture of a 5 % aqueous solution of sodium nitrite (249.3 g) and a 2.5 N aqueous solution of sodium hydroxide (401.6 ml) all at once while stirring.

After the mixture was stirred at room temperature for 10 minutes and at 70° C for 20 minutes, it was adsorbed on Amberlite IR-120B ($H^+$ type, 1000 ml) and washed with water till pH of the washing liquid became neutral (pH = 5 to 6) and eluted with 1N aqueous ammonia.

The eluate was collected fraction by fraction, and the ninhydrine positive fractions were combined and evaporated under reduced pressure to obtain yellowish syrupy concentrate. The concentrate was crystallized from water (50 ml) and ethanol (160 ml). The crystal was recovered by filtration and dried under vacuum to obtain L(-)-4-amino-2-hydroxybutyric acid (IVa) (14.1 g).

The properties of L(-)-4-amino-2-hydroxybutyric acid were as follows:

Melting point: 191-192° C

$[\alpha]_D^{23}$ .5 = -26.5° (c = 2.5 in water)

$^1$H-NMR spectrum: the same as in Example 1.

Reference Example 2

Preparation of 5-methyl L-aspartate (Ib)

In a mixture of methanol (300 ml) and conc. sulfuric acid (31.9 g), L-aspartic acid (30 g) was added and stirred at room temperature overnight. The analysis of the reaction mixture with high performance liquid chromatography (HPLC) revealed that 4-methyl L-aspartate (Ib) (28.3 g, purity of 85.3 %) was formed.

Example 3

Preparation of L-2-hydroxyasparagine (IIIb)

The reaction mixture (175 ml) containing 4-methyl L-aspartate (103.5 mmol) obtained in Reference Example 2 was neutralized with a saturated aqueous solution of sodium bicarbonate to pH of 6.0, and methanol was evaporated off under reduced pressure. Water was added to a whole volume of 100 ml.

To the mixture, acetic acid (28.0 g) was added and then a 40 % (w/w) aqueous solution of sodium nitrite (27.1 g) was added over about 5 hours while stirring at a temperature of 15 to 20 °C and further stirred overnight.

From the reaction mixture, acetic acid was evaporated off under reduced pressure, and the concentrate was acidified to pH of 2.0 with conc. hydrochloric acid and extracted with ethyl acetate (each 50 ml) five times. The combined organic layers were dried over magnesium sulfate, followed by evaporating off ethyl acetate under reduced pressure to obtain a syrupy concentrate (IIb) (13.5 g).

Then, the syrupy concentrate was dissolved in water (20 ml) and gradually poured in an ice-cooled 28 % aqueous ammonia. After pouring, the solution was gradually warmed to room temperature and kept standing overnight, followed by evaporation under reduced pressure to obtain a syrupy L-2-hydroxyasparagine (IIIb).

Then, to the syrup, water (10 ml) was added to form a solution, and the solution was passed through a column containing Amberlite IR-120B resin ($H^+$ type, 200 ml) followed by washing with water. Acidic eluates having pH of not larger than 4.0 (about 700 ml) were collected and evaporated under reduced pressure to obtain white crystalline L-2-hydroxyasparagine (IIIb) (9.2 g, purity of 67.3 %). The compound (IIIb) was dissolved in ethanol (40 ml) by heating and cooled to crystallize it. The crystallized product was recovered by filtration and dried under vacuum.

The properties of L-2-hydroxyasparagine (IIIb) were as follows:

Melting point: 131-133 °C

$[\alpha]_D^{25}$ = -7.47° (c = 25, water).

Thin layer silica gel chromatography (coloring with phosphomolybdic acid): $R_f$ = 0.6

(ethanol:acetic acid:butanol:water = 10:2:2:1)

Example 4

Preparation of L(-)-3-amino-2-hydroxypropionic acid (IVb) (L-isoserine)

To an ice-cooled mixture of a 5 % aqueous solution of sodium hypochlorite (56.0 g) and a 2.5 N aqueous solution of sodium hydroxide (90.0 ml), L-2-hydroxyasparagine (IIIb) obtained in Example 3 (5.0 g, 37.5 mmol) was added while stirring. Then the mixture was stirred at room temperature for 10 minutes and then at 70 °C for 20 minutes and cooled. After the mixture was acidified to pH of 4.0 with conc. hydrochloric acid, it was adsorbed on Amberlite IR-120B resin ($H^+$ type, 400 ml), washed with water till pH of the washing liquid became neutral (pH = 5 to 6) and eluted with 1N aqueous ammonia.

The eluate was collected fraction by fraction, and the ninhydrine positive fractions were combined and evaporated under reduced pressure to obtain yellowish syrupy concentrate. The concentrate was dissolved in water (15 ml). Then, to the solution, ethanol (34 ml) was added to crystallize the product. The crystal was recovered by filtration and dried under vacuum to obtain L(-)-3-amino-2-hydroxy-propionic acid (IVb) (2.9 g, purity of 73.6 %), which was recrystallized from water/ethanol.

The properties of L(-)-3-amino-2-hydroxypropionic acid were as follows:

Melting point: 193-194 °C

$[\alpha]_D^{25}$ = -31.2° (c = 25 in water)

$^1$H-NMR ($D_2O$ + CCl) : δ(ppm) = 4.66-4.40 (1H, m) and 3.60-3.00 (2H, m).

Example 5

In the same manner as in Example 3, a syrupy concentrate (IIb) (12.8 g) was obtained.

Then, the syrupy concentrate was dissolved in water (20 ml) and gradually poured in an ice-cooled 28 % aqueous ammonia. After pouring, the solution was gradually warmed to room temperature and kept standing overnight, followed by evaporation under reduced pressure to obtain a syrupy L-2-hydroxyasparagine (IIIb) (9.7 g, 72.8 mmol) which was confirmed by the high performance liquid chromatograph analysis.

After evaporating off ammonia and water under reduced pressure, a resulting concentrate was added to an ice-cooled mixture of a 5 % aqueous solution of sodium nitrite (108.4 g) and a 2.5 N aqueous solution of sodium hydroxide (174.7 ml) all at once while stirring.

After the mixture was stirred at room temperature for 10 minutes and at 70 °C for 20 minutes, it was acidified to pH of 4.0 with conc. hydrochloric acid. Then, the mixture was absorbed on Amberlite IR-120B ($H^+$ type, 400 ml) and washed with water till pH of the washing liquid became neutral (pH = 5 to 6) and eluted with 1N aqueous ammonia.

The eluate was collected fraction by fraction, and the ninhydrine positive fractions were combined and evaporated under reduced pressure to obtain yellowish syrupy concentrate. The concentrate was dissolved in water (15 ml). To the solution, ethanol (36 ml) was added to crystallize the product. The crystal was recovered by filtration and dried under vacuum to obtain L(-)-3-amino-2-hydroxypropionic acid (IVb) (5.2 g).

The properties of L(-)-3-amino-2-hydroxypropionic acid were as follows:

Melting point: 187-188 °C
$[\alpha]_D^{25}$ = -31.4° (c = 25 in water)
$^1$H-NMR spectrum: the same as in Example 3.

## Claims

**1.** a process for the production of an optically active ω-amino-α-hydroxycarboxylic acid of the formula:

$$\begin{array}{l} HO-CH-(CH_2)_n-NH_2 \\ \quad\;\; | \\ \quad\;\; COOH \end{array} \qquad (IV)$$

wherein n is 1 or 2, which process comprises steps of:

(1) reacting an ω-ester of an α-amino acid of the formula:

$$\begin{array}{l} NH_2-CH-(CH_2)_n-COOR \\ \qquad\;\; | \\ \qquad\;\; COOH \end{array} \qquad (I)$$

wherein n is the same as defined above, and R is a $C_1$-$C_7$ alkyl group or an aralkyl group with nitrous acid under an acidic condition to convert an amino group to an hydroxy group to obtain an ω-alkoxycarbonyl-α-hydroxycarboxylic acid of the formula:

$$\begin{array}{l} OH-CH-(CH_2)_n-COOR \\ \quad\;\; | \\ \quad\;\; COOH \end{array} \qquad (II)$$

wherein R and n are the same as defined above,

(2) reacting the ω-alkoxycarbonyl-α-hydroxycarboxylic acid (II) with ammonia to convert an alkoxycarbonyl group to an amide group to obtain an ω-aminocarbonyl-α-hydroxycarboxylic acid of the formula:

$$\begin{array}{l} OH-CH-(CH_2)_n-CONH_2 \\ \quad\;\; | \\ \quad\;\; COOH \end{array} \qquad (III)$$

wherein n is the same as defined above, and

(3) reacting the ω-minocarbonyl-α-hydroxycarboxylic acid (III) with an active halogen to convert the amido group to an amino group to obtain the ω-amino-α-hydroxycarboxylic acid (IV).

**2.** The process according to claim 1, wherein, in the step (1), the amino group is converted to the hydroxy group with at least one nitrate selected from the group consisting of alkali metal salts and alkaline earth metal salts of nitrous acid in an aqueous medium which is acidified with acetic acid.

3. The process according to claim 1, wherein sodium hypochlorite is used to generate active halogen.

4. The process according to claim 1, wherein the compound (I) is an $\omega$-ester of L-$\alpha$-amino acid.

5. The process according to claim 4, wherein the $\omega$-ester is a methyl ester.